# EUROPEAN PATENT APPLICATION

(11) **EP 2 774 581 A1**
(43) Date of publication of application: **10.09.2014**
(21) Application number: 14150581.8
(22) Date of filing: 09.01.2014
(51) Int. Cl.: A61F 2/36, A61F 2/30

(54) **Anatomic monolithic hip implant system**

(30) Priority: 05.03.2013 US 201313785385
(71) Applicant: Howmedica Osteonics Corp., Mahwah, NJ 07430 (US)
(72) Inventor: Gerges, Justin Joseph, Waldwick, New Jersey 07463 (US); Boucher, Florian, 14880 Colleville Montgomery (FR); Collet, Pascal, 14000 Caen (FR)
(74) Representative: Bridge-Butler, Jeremy

(57) **Abstract**

An anatomic prosthetic femoral implant system has a series of modular part-spherical heads and between 4 and 8 femoral component size groups. Each size group has only four components, two left and two right hip implants. One right and one left component being neutral in version and one right and one left being anteverted at an angle producing a head offset of between 4 and 5 mm from neutral. The head offset remains in this 4-5 mm range through all the size groups in the system. It has been found that a large proportion of the target patient population can be served by such a system.

## Description

This invention relates to a monolithic (one-piece) femoral hip prosthesis and more particularly to a femoral hip stem component having an anatomic shape which produces a line-to-line contact with a prepared femoral medullary canal.

Hip arthroplasty procedures involve the implantation of a prosthetic stem component within a femoral intramedullary canal. A ball-shaped head on the proximal end of the stem cooperates with a socket of an acetabulum to provide for articulation between the femur and the acetabulum. In order to maintain pain-free articulation of the hip joint following implantation of the stem, it is important that the stem be securely fastened in the intramedullary canal. When a press-fit or line-to-line contact is desired with the intramedullary canal, the stem contour should closely match the contour of the prepared intramedullary canal.

The anatomy of the femur varies considerably from one individual to another. Version of the proximal femur refers to the relationship of the axes of the femoral neck to the transcondylar access of the distal femur. Femoral anteversion refers to the condition where the femoral axis is rotated anteriorly with respect to the transcondylar femoral axis, with the femoral head directed anteriorly to the coronal plane of the femur. The anteversion angle is defined as the angle by which the femoral neck deviates forwards from the axis of the femoral condyles, projected on to the horizontal plane, and it measures the anterior rotation of the neck of the femur around the shaft. Methods for *in vivo* measurement of the anteversion angle include the trochanteric prominence angle test and the Hermann biplanar radiological technique, although today the method of choice is considered to be CT. Femoral anteversion along with acetabular anteversion provides inherent stability to the hip joint. Such a femoral component implant is shown in U.S. Patent Nos. 5,139,522; 5,358,534; and 5,762,204, the disclosures of which are incorporated herein by reference.

Previously when the plurality of neck version angles were desired, femoral components with modular necks were provided with the necks at various angles to the coronal plane bisecting the hip stem. However, a modular design has several drawbacks, for example, a large number of different parts must be provided in kit form and because of the modular design, stresses within the neck and the connection location within the proximal femur produce a hip stem less resistant to the forces applied during use. Therefore, it is advantageous to provide a one-piece hip stem with the neck made part of the proximal and distal stem portions during manufacture such as by casting or forging.

By analyzing databases of femur images for a large number (greater than 500) femur images and analyzing sales of modular necks for anatomic femoral implant stems. it was determined that the femoral anteversion showed a bi-modal data distribution (two neck anteversion). The bi-modal distribution was not the distribution that one of ordinary skill in the art would expect. One of ordinary skill would expect that three or more anteversion angles and related head offsets would be required for each stem size in a given patient population As a result of this bi-modal distribution, the patient population can be well served by a one-piece, i.e., monolithic or nonmodular implant neck stem system that offers only two anteverted head offsets for any given size or stem. It was determined that these two angles were 0° (neutral version, i.e., the center of the neck and therefore the head lies in the coronal plane) or an anteversion angle which produces a predetermined anteversion. The amount of anteversion offset from neutral associated with each size of stem is the same and is between 4 and 5mm. The amount determined to be most appropriate is approximately 4.57mm.

Thus, the distances between the center of the trunnion for the neutral stem and the center of the trunnion for the anteverted stem is the same regardless of the size of the rest of the stem. This situation is the result of the prosthetic hip implant stem being anatomic. Anatomic implant stems are designed to more closely fill the anterior portion of the femoral canal as compared to tapered wedge stems (such as Accolade^{®} from Stryker and Taperloc^{®} from Biomet) and as a result require a right and left stem. One design philosophy of an anatomic stem is that the centroid of the stem is at the same location as the centroid of the femoral canal. As a result, any size-specific anteversion changes are accounted for in the design of the proximal portion of the stem. For example, larger sizes of bones would be expected to, in general, have more anteversion. Thus, the anteversion increase that is a function of size in many cases is partially addressed in the design of the proximal stem portion leaving the additional offset for the head center relatively constant from size to size.

Thus, for each size stem there would be four monolithic anatomical hip stems, two of which would be shaped for the left femur and two of which would be anatomically shaped for the right femur with two different anteversion angles (0° and approximately 4.5 to 11 degrees selected to produce a 4 to 5mm head anteversion). Typically there are four to eight stem sizes for a particular hip implant system.

As used herein when referring to bones or other parts of the body, the term "proximal" means close to the heart and the term "distal" means more distant from the heart. The term "inferior" means toward the feet and the term "superior" means toward the head. The term "anterior" means toward the front part or the face and the term "posterior" means toward the back of the body. The term "medial" means toward the midline of the body and the term "lateral" means away from the midline of the body.
Fig. 1 shows an example of an anatomically shaped hip stem of a medium size femoral stem implant showing an overlay of both a neutral and anteverted neck attached to identical proximal and distal left stem portions;
Fig. 2 is a lateral elevation view of the femoral component of Fig. 1 showing the neck angle in the coronal plane;
Fig. 2A is a medial elevation view of femoral component of FIGS. 1 and 2.
Fig. 3 is a top view of the femoral component of Figs. 1 and 2 showing the neutral and anteverted neck for a left hip stem; and
Figs. 4 and 5 show two sets of prosthetic femoral components bearing in size from size 1 to size 8 both with neutral version and anteversion of about 4.57mm between the anteverted stem and the neutral stem.
Fig. 4A is a view from the lateral side of the group of the different size prosthetic femoral implant components shown in Fig. 4 showing the proximal to distal length increasing from one size to the next, as well as the anterior-posterior dimension increasing.

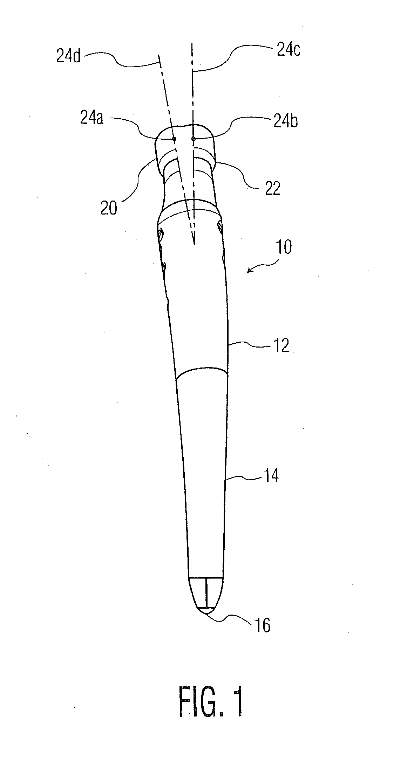

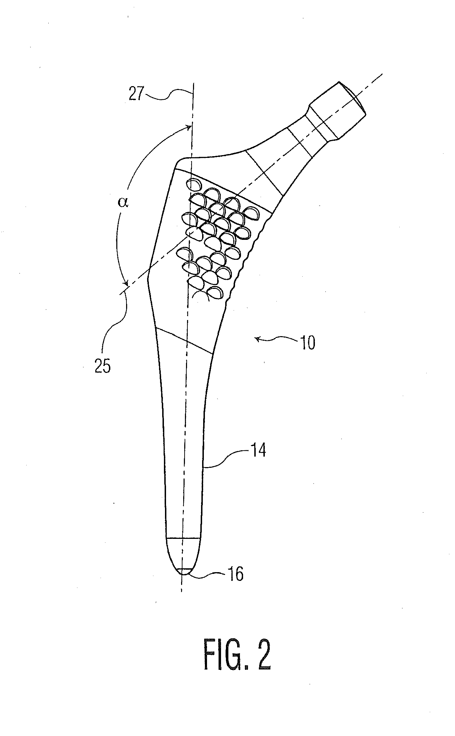

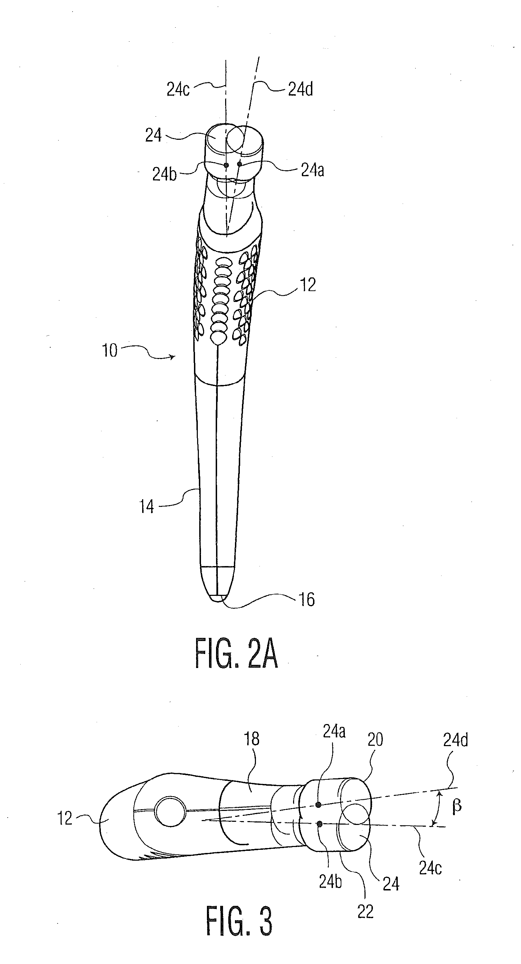

Referring to Fig. 1 and 2A there is shown a left medium size femoral component generally denoted as 10, having a proximal stem portion 12 and distal stem portion 14 with a distal tip 16. Proximally of portion 12, there is shown a neck portion 18 with both an anteverted proximal end 20 and a neutral proximal end 22 with a tapered trunnion 24 for receiving a modular part-spherical head or ball (not shown). The tapered proximal end of the neck 24 may have a Morse taper with a centroid 24a when anteverted and a centroid 24b when neutral. Obviously, for each stem size two separate stems would be provided for both the left and right femoral implant, one having centroid 24b which is neutral in version and the other having centroid 24a which is anteverted a distance 4 to 5 mm from point 24b. Axis 24c is the axis of neck 18 in neutral and axis 24d is the axes of neck 18 when anteverted.

Thus, for each stem size, an anatomic femoral component stem would be provided for the right (not shown) and left femur, each having a distal portion 14 extending along the anterior bow of the femoral shaft with two femoral components provided for the left femur (as shown), one neutral and one anteverted. Typically the anteversion is between 4.5 and 11°, and it has been found from a study of the femur referred to above that an anteversion angle that produced a 4.57mm offset would fit a large portion of the population. It should also be noted that as stem sizes increase or decrease from the medium size shown the anteversion offsets remain relatively constant within groups of patients. The femoral database includes the data that may vary the 4 to 5 mm anteversion offset based on a population of individuals requiring a large or small femoral components. In other words, an analysis of a group of individuals requiring larger femoral components may produce a slightly different medium anteversion (males). This would be likewise for the group of individuals requiring a smaller femoral component (female). Obviously, any number of population groups can be addressed by different size femoral components, however, the population is usually grouped into three to five different groupings (male, female, Asian, etc.).

Referring to Fig. 2, there is shown an elevation view of the femoral component showing a neck axis 25 angle α of 127°, which is the angle between the longitudinal axis 27 of the stem in the coronal plane and the neck axis 25 in the coronal plane, which typically is between 125 and 140°. It has been found by an analysis of the femur database that angles α of 127° and 132° provide the best match for the patient population.

Referring to Fig. 3, there is shown a top or proximal to distal view of the hip implant set of Fig. 1 for a left femoral component with the two neck version angles, one 0° and the anteverted angle β for the component shown in Fig. 3. This anteverted neck angle β produces an anteversion of between 4 and 5mm and most preferably 4.57mm on each size stem. Normally 4 to 8 femoral implant stem sizes are provided which, in an anatomic stem, have proximal and distal portions suitable for use with a large proportion greater than 50% of the male and female patient population being served. By providing only two version angles per stem size neutral (0°) and an angle which produces an anteversion at the center of the trunnion 24a (also the approximate location of the center of a part-spherical head of the prosthetic femoral component) of approximately 4.57mm.

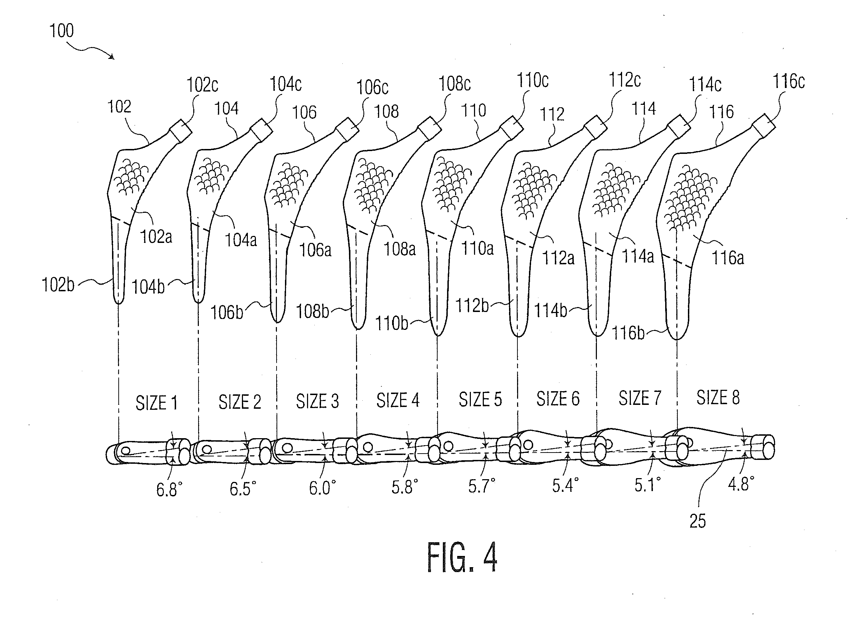

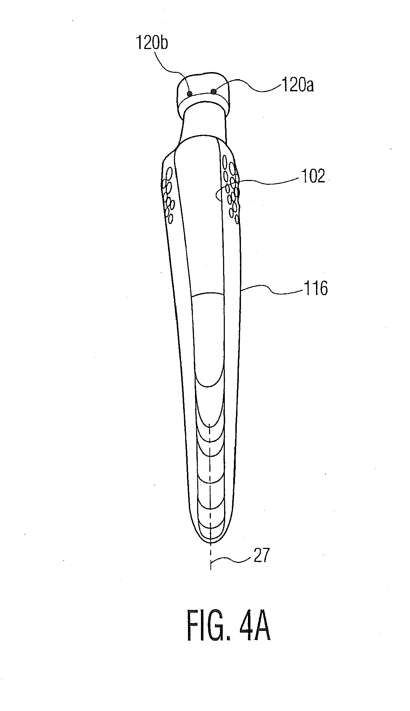

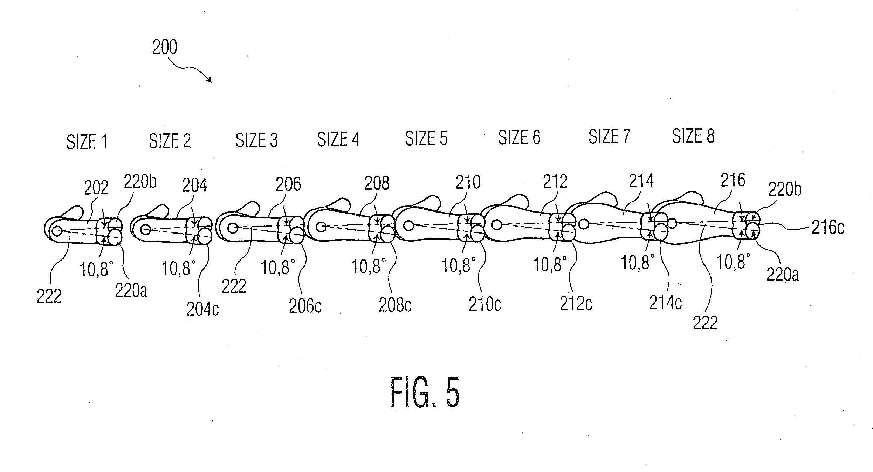

Referring to Figs. 4 and 5, there is shown two sets of femoral components designated as 100 and 200. Each set 100 and 200 comprises eight sizes, although more or fewer sizes may be provided. Each size consists of four stems, two right and two left with one left and right being neutral in version and one left and right being anteverted. Referring to Fig. 4, the set of stems 100 includes eight size stems 102 through, 104, 106, 108, 110, 112, 114, and 116, which increase in all dimensions as the size increases from 102 to 116. As can be seen, the upper elevation view shows that the proximal portion increases in both the medial-lateral direction and as shown in Fig. 4A and, the anterior-posterior direction. Likewise, the distal stem portion 102B through 116B increases in cross-section. The stem length also increases with increasing size. The tapered trunnions 102C through 116C stay the same, so the same modular heads (not shown) may be used. Of course femoral components with non-modular heads may be used two lefts and two rights with the anteversion described above. The top views shown at the bottom of Fig. 4 show one way of achieving the desired 4-5mm offset, which is to measure the angle from the longitudinal axis 27 of the proximal and distal stem portions at the neck base and provide varying angles of anteversion as the size of the stems 102-116 increase to produce the desired 4 to 5 mm offset. As can be seen in Fig. 4 at the bottom, the angle decreases from 6.8° to 4.8° as the sizes increase. This also can be seen in Fig. 4A where the centroids 120A and 120B of all the stems 102 through 116 have the same spacing between them, while the stems shown in overlay increase both in length and anterior/posterior width. While the lateral side view shown in Fig. 4A shows an increase in width in the anterior and posterior directions, it should be noted that the stems increase in size in the medial and lateral directions as shown in the upper elevation view shown in Fig. 4.

Referring to Fig. 5, there is shown a top view of eight stem sizes showing stems 202, 204, 206, 208, 210, 212, 214, and 216. In the design of stems 202 through 216, the spacing between the trunnion centroids remains the same as that shown in Fig. 4, that is between 4 and 5mm, preferably 4.57mm, but with a constant anteversion angle between the neutral stem and the anteverted stem. As shown in Fig. 5, this angle is 10.8° so that the length between the centroid 220A and 220B of the stem 202 remains between 4 and 5mm. As the size increases, the distance between centroid 220A and 220B and the anteversion angle remains constant, however, the origin or apex 222 of the 10.8° angle shifts proximally up the neck toward the trunnion 202C through 216C. Since, as the neck length increases and the angle remains the same as well as the side opposite the origin or apex (head offset of 4 to 5 mm), the length of the side lying along the neck axis for the neutral stem also stays constant, thereby requiring the apex to move proximally along the neck axis 25. It should be noted that all the stems shown in Figs. 4 and 5 are for the left femur and thus a similar set of neutral and anteverted femoral implant components would be provided for the right femur.

Thus, the hip system of the present invention provides four stems, two neutral inversion, and two having an anteversion angle, which shifts the centroid of the trunnion approximately 4 to 5mm from that of the neutral stem so that only four stems per size are required. It has unexpectedly been found that the four stems provided, two for the left and two for the right femur, with one stem being neutral inversion and the other being anteverted as described above, provide adequate choices for covering a large portion of a patient population. Thus, a surgeon performing a total hip replacement on, for example, a right femur has to select an appropriate size monolithic implant and then merely select a neutral or anteverted stem. As indicated above, an anatomic stem design, which produces a line-to-line contact with a prepared femoral canal in combination with the four stems per size described herein provide the desired anteversion for the majority of patients in a given population group.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A prosthetic femoral implant system comprising:
a part-spherical head for mounting on a neck portion of a femoral component;
a first group of four monolithic femoral components each having a distal stem portion, a proximal stem portion and a neck portion, the distal portions, proximal portions, and neck portions of the first group all having the same first length, the proximal and distal stem portions of each femoral component in the first group having a shape conforming to an anatomic shape of a femoral canal, first and second of the four monolithic femoral components of the first group conforming to a right femoral canal and third and fourth of the four monolithic femoral components of the first group confirming to a left femoral canal, the first and third femoral components having a neck locating the head neutral in version and the second and fourth stem have a neck anteverted, when implanted, at an angle producing a head center anteversion of 4 to 5mm; and
a second group of four monolithic femoral components each having a distal stem portion, a proximal stem portion and a neck portion, the distal portions, proximal portions, and neck portions of the second group all having the same second size, the second size being different than the first size, the proximal and distal stem portions of the femoral components of the second group having a shape conforming to an anatomic shape of a femoral canal, first and second femoral components of the four monolithic femoral component of the second group conforming to a right femoral canal and third and fourth femoral components of the second group conforming to a left femoral canal, the first and third femoral components of the second group having a neck being neutral in version and the second and fourth femoral components of the second group having a neck being anteverted, when implanted, at an angle producing a head center anteversion of between 4 to 5mm with respect to the first and third femoral components of the second group.

2. The prosthetic femoral implant system as set forth in claim 1 wherein the anteversion of the second and fourth stems of the first and second groups is between about 4.5 and 11.°.

3. The prosthetic femoral implant system as set forth in claim 1 further comprising a third group of four monolithic femoral components having distal stem portions, proximal stem portions and neck portions having a third size different than the first and second sizes.

4. The prosthetic femoral implant system as set forth in claim 1 wherein a central axis of the neck portion of each of the femoral components forms an angle of 127° with a longitudinal axis of the proximal and distal stem portions.

5. The prosthetic femoral implant system as set forth in claim 4, wherein the system further comprises an additional set of four monolithic femoral components having distal stem portions and proximal stem portions each having the lengths the same as lengths of the first and second groups of four monolithic femoral components but having an angle of 132° between the central axis of the neck portions and the longitudinal axis of the distal and proximal portions.

6. The prosthetic femoral implant system as set forth in claim 5, wherein the anteverted neck portions of the second and fourth stems of the first and second groups are anteverted at between 5 and 11°.

7. The prosthetic femoral implant system as set forth in claim 1, wherein the proximal and distal stem portions of all the stems are shaped to form a line to line fit with a femoral cavity.

8. The prosthetic femoral implant as set forth in claim 1 wherein the anteversion of the second and fourth stems of the first and second groups of stems is 4.57°.

9. A prosthetic femoral implant system comprising:
a first group of four monolithic femoral components each having a distal stem portion, a proximal stem portion and a neck portion with a prosthetic femoral head formed thereon, the distal portions, proximal portions, and neck portions of the first group all having the same first length, the proximal and distal portions of each femoral component in the first group having a shape conforming to an anatomic shape of a femoral canal, first and second of the four monolithic femoral components of the first group conforming to a right femoral canal and third and fourth of the four monolithic femoral components of the first group confirming to a left femoral canal, the first and third femoral components having a head center locating the head neutral in version and the second and fourth stem have the head anteverted, when implanted, at an angle producing a head center anteversion of 4 to 5mm; and
a second group of four monolithic femoral components each having a distal stem portion, a proximal stem portion and a neck portion with a prosthetic head formed thereon, the distal portions, proximal portions, and neck portions of the second group all having the same second size, the second size being different than the first size, the proximal and distal stem portions of the femoral components of the second group having a shape conforming to an anatomic shape of a femoral canal, first and second femoral components of the four monolithic femoral component of the second group conforming to a right femoral canal and third and fourth femoral components of the second group conforming to a left femoral canal, the first and third femoral components of the second group having a head center being neutral in version and the second and fourth femoral components of the second group having a neck being anteverted, when implanted, at an angle producing a head center anteversion of between 4 to 5mm with respect to the first and third femoral components of the second group.

10. The prosthetic femoral implant system as set forth in claim 9 wherein the anteversion of the second and fourth stems of the first and second groups is between about 4.5 and 11.°.

11. The prosthetic femoral implant system as set forth in claim 9 further comprising a third group of four monolithic femoral components having distal stem portions, proximal stem portions and neck portions having a third size different than the first and second sizes.

12. The prosthetic femoral implant system as set forth in claim 9 wherein a central axis of the neck portion of each of the femoral components forms an angle of 127° with a longitudinal axis of the proximal and distal stem portions.

13. The prosthetic femoral implant system as set forth in claim 12, wherein the system further comprises an additional set of four monolithic femoral components having distal stem portions and proximal stem portions each having the lengths the same as lengths of the first and second groups of four monolithic femoral components but having an angle of 132° between the central axis of the neck portions and the longitudinal axis of the distal and proximal portions.

14. The prosthetic femoral implant system as set forth in claim 13, wherein the anteverted neck portions of the second and fourth stems of the first and second groups are anteverted at between 5 and 11°.

15. The prosthetic femoral implant system as set forth in claim 9, wherein the proximal and distal stem portions of all the stems are shaped to form a line to line fit with a femoral cavity.
